# EUROPEAN PATENT SPECIFICATION

(11) **EP 3 213 747 B1**
(45) Date of publication and mention of the grant of the patent: **13.02.2019**
(21) Application number: 16382101.0
(22) Date of filing: 04.03.2016
(51) Int. Cl.: A61K 9/20, A61K 31/10, A23L 33/10, A23L 33/175, A61K 31/198, A61K 31/295, A61K 33/26

(54) **TABLETS WITH A HIGH-CONTENT OF ACTIVE INGREDIENTS USEFUL FOR TREATING HAIR LOSS OR STIMULATING ITS GROWTH**
TABLETTEN MIT HOHEM GEHALT AN WIRKSTOFFEN ZUR BEHANDLUNG VON HAARVERLUST ODER ZUR ANREGUNG IHRES WACHSTUMS
COMPRIMÉS PRÉSENTANT UNE TENEUR ÉLEVÉE EN INGRÉDIENTS ACTIFS UTILES POUR LE TRAITEMENT DE LA PERTE DE CHEVEUX OU LA STIMULATION DE LEUR CROISSANCE

(43) Date of publication of application: 06.09.2017
(73) Proprietor: Laboratorio Reig Jofre S.A., 08970 Sant Joan Despí (ES)
(72) Inventor: GONZÁLEZ PLÁGARO, Jordi, 08970 SANT JOAN DESPÍ (ES); NIETO ABAD, Carlos, 08970 SANT JOAN DESPÍ (ES); BORRÁS SCHIERLOH, José María, 08970 SANT JOAN DESPÍ (ES); JODAR CHACÓN, Jorge, 08970 SANT JOAN DESPÍ (ES); NAVARRO PUJOL, Francisco, 08970 SANT JOAN DESPÍ (ES)
(74) Representative: ZBM Patents - Zea, Barlocci & Markvardsen

(56) References cited:
- WO-A1-03/035027
- CN-A- 104 688 763
- GB-A- 2 314 019
- GB-A- 2 484 812
- JOHN J JANOWIAK ET AL: "A practitioner's guide to hair loss: Part 2 - Diet, supplements, vitamins, minerals, aromatherapy, and psychosocial aspects", ALTERNATIVE AND COMPLEMENTARY THERAPIES, vol. 10, no. 4, 1 January 2004 (2004-01-01), pages 200-205, XP055298076, US ISSN: 1076-2809, DOI: 10.1089/1076280041580431

## Description

The invention relates to compositions having a high drug load useful for the treatment of hair loss.

### BACKGROUND ART

Hair loss is a common and embarrassing disorder that affects men and women of all ages. Hair loss occurs when the cycle of hair growth and shedding is disrupted or when the hair follicle is destroyed and replaced with scar tissue. The exact cause of hair loss may not be fully understood, but it is usually related to one or more of the following factors: age and genetic factors, inadequate nutrition, hormonal imbalance, medical disorders such as cancer and anemia, certain types of medication and chemotherapy, environmental factors such as pollution, trauma and stress, rapid weight loss infections, and improper hair care. Heredity affects the age at which the hair loss begins, the rate of hair loss and the extent of baldness.

Pattern baldness is most common in men and can begin as early as puberty. This type of hair loss may involve both hair thinning and miniaturization, i.e. hair becomes soft, fine and short. Thinning hair and hair loss are also common in women, and no less demoralizing. Reasons can range from a vitamin deficiency or an anemia due to an iron deficiency to more complex reasons like an underlying health condition.

Many of the hair loss remedies available today rely on synthetic chemicals. Such products give only temporary relief. The application of temporary and harmful chemicals is not an ideal scenario for the treatment of hair disorders, which need a solution that can be used over the lifetime of the individual. On the other hand, certain nutritional components can provide some effective, long term, and easy to use remedies, in particular, in those cases where the hair loss is due to vitamins or an iron deficiency.

Several active ingredients are known for being useful to prevent hair loss. Thus, WO2009150421A2 discloses a pharmaceutical composition to prevent hair loss containing methylsulfonylmethane and L-lysine. According to this document the methylsulfonylmethane contains sulfur which is required for the production of keratin and collagen. Both are important constituents of the hair. The L-lysine increments the iron reserve and prevents hair loss.

GB 2314 019 A discloses tablets comprising L-lysine, vitamin C, iron, and vitamin B12 for treating hair loss.

The use of iron salts and of ascorbic acid hair dye products is also known, for instance, CN101812246A discloses hair dye products containing a mordant which contains iron salts and ascorbic acid.

Nutritional supplement formulations containing active ingredients like vitamins, a source of iron, or other useful active ingredients are used to treat such disorders. However, these formulations generally require a high content of active ingredients, which in the case of being formulated as a tablets, result in tablets of considerable size.

Because of the high drug load of active agents needed, the amount of necessary excipients to formulate the tablet is minimized to avoid problems of swallowing the tablet, but then the mechanical properties of the active agents such as compactability predominate in the tablet.

Concerning to the preparation of the tablets, the direct compression technique is generally preferred to wet granulation. The direct compression technique comprises mixing the dry ingredients followed by compressing them into tablets. it reduces the higher costs involved in wet granulation due to the drying step, including equipment, labor, time, process validation and energy expenditure. Furthermore, direct compression can improve the physical and chemical stability of tablets as compared to wet granulation. For these reasons the companies are focusing increasingly on this process. Unfortunately, a formulation with a high content of active ingredients cannot be easily prepared by direct compression because of the poor flowability and a poor compressibility of the mixture due to the mechanical properties of the active ingredients. This results in tablets that suffer the effect of "capping" i.e. exfoliation and weight variability.

On the other hand, compositions formulated as tablets are generally coated to facilitate swallowing and have a more aesthetic appearance. To be coated, the core must be hard enough to withstand the coating process, must have low friability and be absence of a capping effect.

Thus, the development of a technique for direct compression, and in particular for high drug load tablets, is more challenging that for wet granulation. It is more difficult to achieve homogeneous mixing and a free-flowing mixture in order to obtain tablets with acceptable hardness and friability. Furthermore, direct compression demands the use of excipients with strictly defined properties.

Microcrystalline cellulose has been widely used as an excipient for direct compression. Microcrystalline cellulose has also been used as an excipient of tablets with a high content of active ingredient. Thus, for instance, WO9942087A discloses pharmaceutical compositions with a high content of active ingredient (at least 70% w/w of KCI) in the form of coated pellets, where the majority excipient is microcrystalline cellulose (10-25% w/w). The microcrystalline cellulose used is AVICEL PH105 which has a medium particle size of 20 µm.

Despite what is known in the art, there is still the need of providing a formulations having the high content of active ingredients needed for the prevention of hair loss that can be administered in the form of a tablet prepared by direct compression with small amounts of excipients since the amount of active ingredients is already high for a tablet. It should also be suitable for coating.

### SUMMARY OF THE INVENTION

Inventors have developed a tablet comprising the high load of active ingredients which are needed to treat hair loss that can be prepared by direct compression despite the high content of active ingredients and where the core has the appropriate characteristics which allow further coating. This goal is achieved thanks to the use of L-lysine with a heterogeneous particle size distribution which surprisingly improves the compactability of the mixture, and also because of the specific selection of components and their amounts.

These good properties concerning good compressibility are not obtained when an L-lysine with a more homogenous particle size distribution is used, despite the fact that a priori, it would be considered to be more appropriate for a direct compression process.

Concerning to the excipients, the specific microcrystalline cellulose used as a diluent in the formulation of the invention contributes to obtain a good compressibility. It is characterized by its huge particle size (medium particle size of 180 µm-250 µm). A priori a high particle size would imply low values of hardness. Contrary, to what would be expected the direct compression of the composition of the invention using microcrystalline cellulose of grade 200 works surprisingly well in terms of compressibility and of obtaining good values of hardness, friability and disgregation time.

Thus, an aspect of the present invention relates to an orally administrable composition in the form of a tablet obtainable by direct compression, which comprises a therapeutically effective amount of a combination of active ingredients and edible acceptable excipients, wherein, the total amount of the active ingredients is equal to or higher than 50% of the weight of the tablet core composition; the combination of active ingredients comprises at least: a) methylsulfonylmethane which is present in an amount within the range of from 5 to 40% by weight in respect of the weight of the tablet core composition, b) L-lysine or a salt thereof in the form of particles, which is present in an amount within the range of from 10 to 40% by weight in respect of the weight of the tablet core composition and wherein 40-70% by weight of the particles have a particle size ≥400 µm and 60-85% by weight of the particles has a particle size ≥ 250 µm; c) ferrous fumarate which is present in an amount within the range of from 2 to 20% by weight in respect of the weight of the tablet core composition, and the at least one edible acceptable excipient comprises: d) microcrystalline cellulose in the form of particles, which is present in an amount within the range of from 20 to 50% by weight in respect of the weight of the tablet core composition and wherein up to a 5% by weight of the particles have a particle size ≥400 µm, at least 10% by weight of the particles have a particle size ≥ 250 µm; and at least 50% by weight of the particles have a particle size ≥ 150 µm; e) at least one lubricant; and f) at least one disintegrant.

Optionally, the tablets comprise ascorbic acid which is present in an amount within the range of from 1 to 10% by weight in respect of the weight of the tablet core composition.

Advantageously, a mixture of the components of the previous composition with the features mentioned above can be formulated in the form of tablet cores by direct compression without adding water or any other solvent. Therefore, the direct compression technique is fast, involves only one step prior to compression and includes no solvents, so degradation of the active ingredients during the processing is reduced to a large extent.

Another aspect of the present invention relates to a composition as defined above, for use for treating hair loss and/or stimulating its growth in a subject in need thereof.

### DETAILED DESCRIPTION OF THE INVENTION

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

The expression "high active ingredient content" or "high load of active ingredient" has been indistinctively used to describe an amount of active ingredient in a tablet that is higher than would normally be attainable without using the process and components described herein.

The composition of the present invention may be a nutritional supplement.

The term "dietary supplement", "food supplement" or "nutritional supplement" as used herein interchangeably refers to a preparation intended to supplement the diet and provide nutrients, such as vitamins, minerals, fiber, fatty acids, or amino acids among others, that may be missing or may not be consumed in sufficient quantity in a person's diet.

The expression "therapeutically effective amount" as used herein, refers to the amount of active ingredient that, when administered, is sufficient to prevent development of, or alleviate to some extent, one or more of the symptoms of the condition which is addressed. The particular dose of active ingredient administered according to this invention will of course be determined by the particular circumstances surrounding the case.

The expression "edible acceptable excipients or carriers" refers to edible acceptable materials, compositions or vehicles. Each component must be acceptable in the sense of being compatible with the other ingredients of the composition. It must also be suitable for use for humans and animals with a reasonable benefit/risk ratio.

For the purposes of the invention, any ranges given include both the lower and the upper end-points of the range. Ranges given should be considered approximate, unless specifically stated.

The amount ranges of each of the components of the composition are given with respect to the weight of the uncoated tablet or tablet core composition. Thus, in case the tablet is coated, the amounts of the components given refer to the core of the tablet.

As mentioned above, the composition of the present invention contains a high load of active ingredients. This results in a high weight of the tablet. Generally, the weight of the tablet is between 0.6-1.6g. Preferably, the weight of the uncoated tablet is 1.4 g. Also more preferably, the weight of the coated tablet is 1.5 g.

All the embodiments mentioned above or below are also disclosed optionally in combination with one or more features of the other embodiments defined below or above,

The total amount of active ingredients in the tablet of the present invention is equal to or higher than 50% by weight with respect to the weight of the tablet core composition. In a preferred embodiment, the total amount of active ingredients is equal to or higher than 55% of the weight of the tablet core composition. In a more preferred embodiment, the total amount of active ingredients is equal to or higher than 58% of the weight of the tablet core composition. In another preferred embodiment, the total amount of active ingredients is within the range of 50-75% of the weight of the tablet core composition. In another more preferred embodiment, the total amount of active ingredients is within the range of 50-65% of the weight of the tablet core composition. In a still more preferred embodiment, the total amount of active ingredients is within the range of 50-60% of the weight of the tablet core composition.

The L-Lysine in the composition of the present invention is in the form of particles wherein 40-70% by weight of the particles have a particle size ≥400 µm and 60-85% by weight of the particles have a particle size ≥ 250 µm. The particle size determinations of the L-lysine have been carried out by sieve analysis. The fact that 40-70% by weight of the particles are retained in a sieve having a pore size of 400 µm, means that 30-60% by weight of the particles have a particle size lower than 400 µm and the fact that 60-85% by weight of the particles are retained in a sieve having a pore size of 250 µm means that 15-40% by weight of the particles have a particle size lower than 250 µm.

In a preferred embodiment, the L-Lysine in the composition of the present invention is in the form of particles, wherein 44-60% by weight of the particles of the L-Lysine have a particle size ≥400 µm and 68-78% by weight of the particles have a particle size ≥ 250 µm. In a more preferred embodiment, the L-Lysine in the composition of the present invention is in the form of particles, wherein 45-51 % by weight of the particles of the L-Lysine have a particle size ≥400 µm and 70-76% by weight of the particles have a particle size ≥ 250 µm.

Sieve analysis assumes that all particles will be round or nearly so and will pass through the square openings when the particle diameter is less than the size of the square opening in the screen. A typical sieve analysis involves a nested column of sieves with wire mesh cloth (screen). A representative weighed sample is poured into the top sieve which has the largest screen openings. Each lower sieve in the column has smaller openings than the one above. At the base is a round pan, called the receiver. The column is typically placed in a mechanical shaker. The shaker shakes the column, usually for some fixed amount of time. After the shaking is complete the material on each sieve is weighed. The weight of the sample of each sieve is then divided by the total weight to give a percentage retained on each sieve.

The results are presented as the percent of particles passing through each sieve. First it is calculated the percent retained in each sieve and then the cumulative percent of aggregate retained in each sieve can be found by adding up the total amount of particles that are retained in each sieve and the amount in the previous sieves. The cumulative percent passing of the aggregate is found by subtracting the percent retained from 100%. The method employed is as disclosed in the European Pharmacopeia, monograph 2.9.38 (01/2010). Particle size distribution estimation by analytical sieving.

The L-lysine is present in the composition in an amount within the range of from 10 to 40% by weight in respect of the weight of the tablet core composition. In a preferred embodiment, the amount of L-lysine in the composition of the present invention is within the range of from 21 to 27% by weight in respect of the weight of the tablet core composition. In a more preferred embodiment, the amount of L-lysine in the composition of the present invention is within the range of from 26 to 27% by weight in respect of the weight of the tablet core composition.

The L-lysine may be in the form of an acceptable salt for human use . The term acceptable salts for human use" used herein encompasses any salt formed from acceptable non-toxic acids including inorganic or organic acids. There is no limitation regarding the salts, except that they must be appropriate for human use (pharmaceutical use, edible use, etc.). Preferably, the acceptable salt is the hydrochloride salt.

The methylsulfonylmethane is present in the composition in an amount within the range of from 5 to 40% by weight in respect of the weight of the tablet core composition. In a preferred embodiment, the amount of methylsulfonylmethane in the composition of the present invention is within the range of from 15 to 25% by weight in respect of the weight of the tablet core composition. In a more preferred embodiment, the amount is within the range of from 17 to 22% by weight in respect of the weight of the tablet core composition. In a still more preferred embodiment, the amount is within the range of from 21 to 22% by weight in respect of the weight of the tablet core composition.

The ferrous fumarate is present in the composition in an amount within the range of from 2 to 20% by weight in respect of the weight of the tablet core composition. In a preferred embodiment, the amount of ferrous fumarate is within the range of from 2 to 8% by weight in respect of the weight of the tablet core composition. In another more preferred embodiment, the amount of ferrous fumarate is within the range of from 4 to 6.5% by weight in respect of the weight of the tablet core composition. In a still another more preferred embodiment, the amount of ferrous fumarate is 6% by weight in respect of the weight of the tablet core composition.

The composition of the present invention may further comprise additional active ingredients. In a particular embodiment, it comprises biotine as additional active ingredient in an amount which is in the range of 0.001 to 0.01 % by weight in respect of the weight of the tablet core composition.

The material to be compressed into a tablet according to the present invention comprises several excipients, which help to impart the free-flowing, lubrication, and cohesive properties to the active ingredients that are formulated as a tablet. Thus, the composition of the invention comprises several edible acceptable excipients which at least are the following: microcrystalline cellulose, at least one lubricant, and at least one disintegrant.

The microcrystalline cellulose in the composition is present in an amount which is in the range of 20 to 50% by weight in respect of the weight of the tablet core composition. In a preferred embodiment, the amount of microcrystalline cellulose is within the range of 29 to 41% by weight in respect of the weight of the tablet core composition. In a more preferred embodiment, the amount is within the range of 35 to 40% by weight in respect of the weight of the tablet core composition. In a still more preferred embodiment, the amount is within the range of 38 to 39% by weight in respect of the weight of the tablet core composition.

The microcrystalline cellulose in the composition of the present invention is microcrystalline cellulose grade 200, which is commercial available. According to the Handbook of Pharmaceutical excipients, 6th Edition, 2009 by Raimon C. Rowe, PJ Sheskey, and ME Quin, p129-132, this grade of microcrystalline cellulose has a nominal mean particle size of 180 µm, and has an amount equal to or higher than 10% of particles that are retained in a 60 mesh size sieve (equivalent to 250 µm), and an amount equal to or higher than 50% of particles that are retained in a 100 mesh size sieve (equivalent to 149 µm). This means that at least 10% by weight of the particles have a particle size ≥ 250 µm, and that at least 50% by weight of the particles have a particle size ≥ 150 µm.

Lubricants are preferably selected from the group consisting of magnesium stearate, magnesium lauryl sulfate, sodium stearyl fumarate, sucrose esters or fatty acids, polyethylene glycol, stearic acid, and mixtures thereof. In a preferred embodiment the lubricant is magnesium stearate. Generally, the lubricant is present in an amount ranging from 0.25 to 5% by weight of the tablet core composition.

Disintegrants have an important role in the disintegration and the dissolution process of tablets. The composition of the invention comprises at least one disintegrant for such purpose. Disintegrants are preferably selected from the group consisting of croscarmellose sodium (cross linked carboxymethyl cellulose sodium), crospovidone (cross linked polyvinylpyrrolidone), sodium starch glycolate, corn starch, potato starch, maize starch, and modified starches calcium silicates, low substituted hydroxypropylcellulose, and mixtures thereof. In a preferred embodiment, the disintegrant is croscarmellose sodium. Generally, the disintegrant is present in the composition in an amount of 0.5 to 15% by weight of the tablet core composition.

The composition of the invention may also comprise other excipients such as diluents, glidants, antioxidants, or binders.

Apart from the microcrystalline cellulose, examples of suitable diluents are anhydrous lactose, lactose monohydrate, dibasic calcium phosphate, tribasic calcium phosphate, silicified microcrystalline cellulose, powdered cellulose, spray dried mixtures of lactose and microcrystalline cellulose (commercially available), maize starch, pregelatinized starch, calcium carbonate, sucrose, glucose, dextrates, dextrins, dextrose, fructose, lactitol, mannitol, sorbitol, and/or mixtures thereof. It is preferred to use diluents of direct compression grade.

Examples of suitable glidants are silicon dioxide, talc and aluminium silicate. In a preferred embodiment, the glidant is present and is talc.

Examples of suitable antioxidants are vitamin E acetate, α-tocopherol, ascorbil palmitate, butylated hydroxyanisole, butylated hydroxytoluene, and mixtures thereof.

Examples of suitable binders are polyvinylpyrrolidone, starches such as pregellatinized starch or plain starch, cellulose derivatives such as hydroxypropylmethylcellulose, hydroxypropylcellulose and carboxymethylcellulose, and mixtures thereof.

In addition to the above, the compositions may contain further additives such as sweeteners, flavorings, coloring agents, mixtures thereof, and any other suitable component known to the skilled in the art.

In a particular preferred embodiment, the composition of the invention is that wherein the amount of methylsulfonylmethane is within the range of from 21 to 22% by weight in respect of the weight of the tablet core composition, the amount of L-lysine or a salt thereof is within the range of from 26 to 27% by weight in respect of the weight of the tablet core composition, the amount of ferrous fumarate is within the range of from 6% by weight in respect of the weight of the tablet core composition, the amount of ascorbic acid is within the range of from 3 to 4% by weight in respect of the weight of the tablet core composition the amount of biotine is in the range of 0.005 to 0.006% by weight in respect of the weight of the tablet core composition, and the amount of microcrystalline cellulose is in the range of 35-40% by weight in respect of the total weight of the composition.

The capacity of compressibility of the mixture used for the obtention of the tablets is a key feature in the quality of tablets. This feature affects the farmacotechnique properties of the tablet such as hardness, friability, and disgregation. For the determination of these parameters, the methods disclosed in the European Pharmacopeia have been used. Thus, the determination of the hardness of the tablets has been done according to what is established in the European Pharmacopeia, monograph 2.9.8 (01/2008); the determination of the friability has been done according to what is established in the European Pharmacopeia, monograph 2.9.7 (01/2010); and the determination of the tablet disgregation has been done according to what is established in the European Pharmacopeia, monograph 2.9.1 (04/2011).

The tablets of the present invention may be coated, that is, they may contain an outer coating. The coating allow distinctive coloring and can enhance the stability of the tablets. In a particular embodiment, the outer coating is a film coating. A film coating disintegrates in the stomach and/or intestine after swallowing.

Examples of compounds suitable to coat the tablets are sugars, hydroxypropylmethylcellulose, hydroxypropylcellulose, methylcellulose, ethylcellulose, polyvinyl alcohol, sodium carboxymethyl cellulose, coatings based on methacrylic acid such as Eudragits®, and starch based coatings.

The coating is applied in an amount of 3 % to 20% by weight based on the total weight of the coated tablet, preferably of 8% to 12% by weight of the total coated tablet. More preferably, the amount is 8.5-9.5% by weight based on the total weight of the coated tablet.

The tablets may be prepared by a process comprising: a) dry blending the active ingredients and the excipients; and b) compressing the dry mixture into tablets on a tablet press. In a particular embodiment, first the active ingredients are mixed with the microcrystalline cellulose, and then the rest of excipients are added. Blending can be carried out in different mixers known in the art. The compression step may be carried out in a eccentric tabletting machine using tabletting tools for biconcave or flat tablets with or without breaking-line and whose shape can be round (7 to 12 mm in diameter) to elongated (16x8 mm until 24x11 mm).

Subsequently to step b) the tablets may be subjected to a coating step. In a particular embodiment, the tablets are subjected to a film coating step by use of a coating pan.

Preferably, all process steps are carried out in a controlled atmosphere, for instance, under la low moisture, oxygen, and temperature.

It is also part of the invention the composition as defined above for use for treating hair loss or stimulating its growth in a subject in need thereof. Preferably, the subject is a woman. More preferably, the woman has levels of iron and/or ferritine below the normal levels.

This aspect may also be formulated as the use of a composition as defined above for the manufacture of a composition for the treatment of hair loss and/or for stimulating its growth in a subject in need thereof. The present invention also relates to a method of hair loss and/or for stimulating its growth, comprising administering a therapeutically effective amount of the composition as defined above, in a subject in need thereof, in particular, the subject is a woman.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Coated tablets

### Example 1A:

**Table 1A: Tablet composition**

| **Tablet core composition** | **Weight (mg)** | **% w/w with respect to the core** |
|---|---|---|
| Methylsulfonylmethane | 300 | 21.4 |
| L-Lysine hydrochloride (equivalent to 300 mg of L-lysine base per tablet) | 375.2 | 26.8 |
| Ferrous fumarate (equivalent to 28 mg of iron per tablet) | 85.2 | 6.1 |
| Coated Ascorbic acid (equivalent to 50 mg of uncoated ascorbic acid per tablet) | 51.8 | 3.7 |
| Biotine | 0.075 | 0.0053 |
| Microcrystalline cellulose, grade 200 | 538.7 | 38.5 |
| AcDiSol (microcrystalline cellulose) | 28 | 2.0 |
| Magnesium stearate | 7.0 | 0.5 |
| Talc | 14 | 1.0 |
| Total | 1400 | 100 |

| **Coating layer** | **Weight (mg)** | **% w/w with respect to the coated tablet** |
|---|---|---|
| **Coating layer** | 140 | 9.1 |
| **Coated tablet** | 1540 | |

### Example 1B:

**Table 1B: Tablet composition**

| **Tablet core composition** | **Weight (mg)** | **% w/w with respect to the core tablet** |
|---|---|---|
| Methyl-sulfonyl-methane | 400.00 mg | 28.6 |
| L-lysine hydrochloride | 501.80 mg | 35.9 |
| Ferrous fumarate | 106.50 mg | 7.6 |
| Ascorbic acid | 60.00 mg | 4.3 |
| Folic acid | 0.15 mg | 0.011 |
| Cianocobalamine | 0.002 mg | 0.0001 |
| Microcrystalline cellulose, grade 200 | 280.80 mg | 20.08 |
| AcDiSol (microcrystalline cellulose) | 28.00 mg | 2.0 |
| Magnesium stearate | 7.00 mg | 0.5 |
| Talc | 14.00 mg | 1.0 |
| **Total** | 1398.25 | |

| **Coating layer** | **Weight (mg)** | **% w/w with respect to the coated tablet** |
|---|---|---|
| Hypromellose | 38.22 mg | |
| Microcrystalline cellulose | 5.88 mg | |
| Stearic acid | 5.88 mg | |
| Titanium dioxide | 8.82 mg | |
| **Coating layer** | 58.8 | 4 |
| **Coated tablet** | 1457.05 | |

**Table 2: Particle size L-lysine hydrochloride based on sieve analysis**

| **Particle size (µm)** | **%** |
|---|---|
| <500 | near to 60-65 |
| <400 | near to 50-55 |
| <250 | near to 25-30 |
| <180 | near to 10-15 |
| <100 | near to 3-5 |

The tablet core compositions were prepared by first mixing the different active ingredients in a mixer and then compressing the mixture e into tablets on a tabletting using biconcave tabletting tools.

The tablets are subjected to a film coating step by use of a coating pan.

### Examples 2-9: Effect of the particle size of the L-Lysine in the compressibility of the tablets according to the invention

**Table 3: Tablet core composition composition for examples 2-9**

| **Tablet core composition** | **Weight (mg)** | **% w/w with respect to the core** |
|---|---|---|
| Methylsulfonylmethane | 300 | 21.4 |
| L-Lysine hydrochloride (equivalent to 300 mg of L-lysine base per tablet) with the particle size distribution shown | 375.2 | 26.8 |
| Ferrous fumarate (equivalent to 28 mg of iron per tablet) | 85.2 | 6.9 |
| Ascorbic acid (equivalent to 50 mg of uncoated ascorbic acid per tablet) | 51.8 | 3.7 |
| Biotine | 0.075 | 0.0054 |
| Microcrystalline cellulose, grade 200 | 538.7 | 38.5 |
| AcDiSol | 28 | 2.0 |
| Magnesium stearate | 7.0 | 0.5 |
| Talc | 14 | 1.0 |
| Total | 1400 | |

**Table 4: Particle size L-Lysine of Examples 2-9 based on sieve analysis.**

| Examples | L-Lysine hydrochloride particle size (µm) | | | | | Observations during the compressibility |
|---|---|---|---|---|---|---|
| | <500 | <400 | <250 | <180 | <100 | |
| 2 | 64% | 53% | 30% | 13% | 3% | No problems detected |
| 3 | 60% | 49% | 24% | 11% | 3% | No problems detected |
| 4 | 62% | 51% | 27% | 12% | 3% | No problems detected |
| 5 | 62% | 51% | 27% | 12% | 3% | No problems detected |

| Comparative Examples | | | | | | |
|---|---|---|---|---|---|---|
| 6 | 45% | 23% | 8% | 2% | 0% | Capping effect and lamination problems |
| 7 | 52% | 26% | 12% | 4% | 1% | Capping effect and lamination problems |
| 8 | 45% | 23% | 8% | 2% | 0% | Capping effect and lamination problems |
| 9 | 52% | 26% | 12% | 4% | 1% | Capping effect and lamination problems |

The tablet core compositions of Examples 2-9 were prepared in the same way than the compositions of Example 1. The different tablets obtained were analyzed. Table 5 includes the results of the farmacotechnique properties of the tablets hardness, friability, and disgregation. For the determination of these parameters, the methods disclosed in the European Pharmacopeia have been used, in particular the European Pharmacopeia, monograph 2.9.8 (01/2008); the European Pharmacopeia, monograph 2.9.7 (01/2010) ; and the European Pharmacopeia, monograph 2.9.1 (04/2011) respectively.

For the determination of the particle size, the method disclosed in the European Pharmacopeia, monograph 2.9.38 has been used.

**Table 5: Tablet characterization**

| | Hardness | Friability | | Disgregation time (recommended 3-6') |
|---|---|---|---|---|
| | | Weight loss (Recommended ≤0.5%) | Aspect | |
| Examples | | | | |
| 2 | 271 N | 0.27% | Compliance | 4.5 min |
| 3 | 269 N | 0.30% | Compliance | 5.2 min |
| 4 | 265 N | 0.22 % | Compliance | 6.0 min |
| 5 | 269 N | 0.24% | Compliance | 4.7 min |

| Comparative Examples | | | | |
|---|---|---|---|---|
| 6 | 162 N | 0.72% | Non-compliant | 30 s |
| 7 | 144 N | 0.60% | Non-compliant | 42 s |
| 8 | 158 N | 0.64% | Non-compliant | 40 s |
| 9 | 150 N | 0.62% | Non-compliant | 38 s |

The results show that the L-lysine hydrochloride batches of Examples 2-5 show better properties to the ones of Examples 6-9. In particular, the compositions of Examples 2-5 show higher hardness, lower friability values, higher disgregation times and no compressibility problems.

### Comparative Example 10-12: Effect of the presence of microcrystalline cellulose grade 200

The amount of diluent in these examples is lower than the one claimed. However, they illustrate the contribution of the cellulose microcrystalline grade 200 in the compressibility of the tablet of the invention compared to calcium hydrogen phosphate.

**Tablet 6: Composition of the tablet**

| **Tablet core composition** | Comparative Example 10 | Comparative Example 11 | Comparative Example 12 |
|---|---|---|---|
| Methylsulfonylmethane | 28.57% | 28.57% | 28.57% |
| L-lysine hydrochloride | 35.84% | 35.84% | 35.84% |
| Ferrous fumarate | 9.78% | 9.78% | 9.78% |
| Coated Vitamin C | 4.42% | 4.42% | 4.42% |
| Folic acid | 0.011% | 0.011% | 0.011% |
| Vitamin B-12 | 0.0001% | 0.0001% | 0.0001% |
| Mycrocrystalline cellulose grade 200 | 17.9% | 5.0% | --- |
| Calcium hydrogen phosphate (Emcompress ®) | --- | 12.9 | 17.9% |
| Croscarmellose sodium | 2.00% | 2.00% | 2.00% |
| Magnesium stearate | 0.50% | 0.50% | 0.50% |

**Table 7: Tablet characterization**

| | Hardness | Friability | | Disgregation time |
|---|---|---|---|---|
| | | Weight loss | Aspect | |
| Comparative Examples | | | | |
| 10 | 224 N | 0.36% | Compliance | 4.5 min |
| 11 | 235 N | 0.44% | Compliance | 5.2 min |
| 12 | 150 N | 0.62 % | Compliance | 6.0 min |

The previous comparative examples show the effect on the properties of the tablets of the cellulose microcrystalline used which has a higher particle size than the one used in WO9942087A, and also it has been compared to the use of another diluent (calcium hydrogen phosphate).

The results show that the use of this specific microcrystalline cellulose has a contribution in higher hardness and lower friability values obtained with the composition of the invention.

### REFERENCES CITED IN THE APPLICATION

- WO2009150421A2
- CN101812246A
- WO9942087A
- Handbook of Pharmaceutical Excipients 6th Edition, 2009 by Raimon C. Rowe PJ Sheskey and ME Quin, p. 129-133

## Claims

1. An orally administrable composition in the form of a tablet obtainable by direct compression, which comprises a therapeutically effective amount of a combination of active ingredients and edible acceptable excipients,
wherein,
the total amount of the active ingredients is equal to or higher than 50% of the weight of the core tablet composition;
the combination of active ingredients comprises at least:
a) methylsulfonylmethane which is present in an amount within the range of from 5 to 40% by weight in respect of the weight of the tablet core composition;
b) L-lysine or a salt thereof in the form of particles, which is present in an amount within the range of from 10 to 40% by weight in respect of the weight of the tablet core composition, and wherein 40-70% by weight of the particles have a particle size ≥400 µm and 60-85% by weight of the particles has a particle size ≥ 250 µm;
c) Ferrous fumarate which is present in an amount within the range of from 2 to 20% by weight in respect of the total weight of the tablet core composition, and
the edible acceptable excipient comprises:
d) microcrystalline cellulose in the form of particles, which is present in an amount within the range of from 20 to 50% by weight in respect of the weight of the tablet core composition, and wherein up to a 5% by weight of the particles have a particle size a particle size ≥400 µm, at least 10% by weight of the particles have a particle size ≥ 250 µm; and at least 50%by weight of the particles have a particle size ≥ 150 µm;
e) at least one lubricant; and
f) at least one disintegrant.

2. The composition according to claim 1, further comprising another diluent.

3. The composition according to any of the claims 1-2, which is a nutritional supplement.

4. The composition according to any of the claims 1-3, which is a tablet of 0.6-1.6 g.

5. The composition according to any of the claims 1-4, wherein 44-60% by weight of the particles of the L-Lysine have a particle size ≥400 µm and 68-78% by weight of the particles has a particle size ≥ 250 µm.

6. The composition according to any of the claims 1-5, wherein 45-51% by weight of the particles of the L-Lysine have a particle size ≥400 µm and 70-76% by weight of the particles has a particle size ≥ 250 µm.

7. The composition according to any of the claims 1-6, wherein the amount of methylsulfonylmethane is within the range of from 17 to 22% by weight in respect of the weight of the tablet core composition; the amount of L-lysine or a salt thereof is within the range of from 21 to 27% by weight in respect of the weight of the tablet core composition; and the amount of ferrous fumarate is within the range of from 2 to 8% by weight in respect of the weight of the tablet core composition.

8. The composition according to any of the claims 1-7, further comprising biotine as active ingredient in an amount which is in the range of 0.001 to 0.01% by weight in respect of the weight of the tablet core composition.

9. The composition according to any of the claim 1-8, wherein the lubricant is magnesium stearate.

10. The composition according to any of the claims 1-9, wherein the disintegrant is croscarmellose sodium.

11. The composition according to any of the claims 1-10, wherein the amount of methylsulfonylmethane is within the range of from 21 to 22% by weight in respect of the weight of the tablet core composition, the amount of L-lysine or a salt thereof is within the range of from 26 to 27% by weight in respect of the weight of the tablet core composition, the amount of ferrous fumarate is within the range of from 6% by weight in respect of the weight of the tablet core composition, the amount of ascorbic acid is within the range of from 3 to 4% by weight in respect of the weight of the tablet core composition the amount of biotine is in the range of 0.005 to 0.006% by weight in respect of the I weight of the tablet core composition, and
the amount of microcrystalline cellulose is in the range of 35-40% by weight in respect of the weight of the tablet core composition.

12. The composition according to any of the claims 1-11, which further comprises an outer coating.

13. A composition as defined in any of the claims 1-12, for use for treating hair loss and/or stimulating its growth in a subject in need thereof.

14. The composition for use according to claim 13, wherein the subject is a woman.

## Patentansprüche

1. Eine oral verabreichbare Zusammensetzung in Form von einer Tablette, die durch Direktverpressung erhalten werden kann, welche eine therapeutisch wirksame Menge von einer Kombination von Wirkstoffen und essbaren akzeptablen Hilfsstoffen umfasst,
wobei
die Gesamtmenge der Wirkstoffe 50 Gew.-% der Kernzusammensetzung der Tablette oder mehr beträgt;
die Kombination von Wirkstoffen mindestens folgendes umfasst:
a) Methylsulfonylmethan, welches in einer Menge im Bereich von 5 bis 40 Gew.-% bezogen auf das Gewicht der Kernzusammensetzung der Tablette vorhanden ist;
b) L-Lysin oder ein Salz davon in Form von Partikeln, welches in einer Menge im Bereich von 10 bis 40 Gew.-% bezogen auf das Gewicht der Kernzusammensetzung der Tablette vorhanden ist, und wobei 40-70 Gew.-% der Partikel eine Partikelgröße ≥ 400 µm haben und 60-85 Gew.-% der Partikel eine Partikelgröße ≥ 250 µm haben;
c) Eisen(II)-fumarat, welches in einer Menge im Bereich von 2 bis 20 Gew.-% bezogen auf das gesamte Gewicht der Kernzusammensetzung der Tablette vorhanden ist, und wobei der essbare akzeptable Hilfsstoff folgendes umfasst:
d) Mikrokristalline Cellulose in Form von Partikeln, welche in einer Menge im Bereich von 20 bis 50 Gew.-% bezogen auf das Gewicht der Kernzusammensetzung der Tablette vorhanden ist, und wobei bis 5 Gew.-% der Partikel eine Partikelgröße ≥ 400 µm haben, mindestens 10 Gew.-% der Partikel eine Partikelgröße ≥ 250 µm haben; und mindestens 50 Gew.-% der Partikel eine Partikelgröße ≥ 150 µm haben;
e) mindestens einen Schmierstoff; und
f) mindestens einen Zerfallsbeschleuniger.

2. Die Zusammensetzung nach Anspruch 1, weiterhin umfassend einen weiteren Streckmittel.

3. Die Zusammensetzung nach einem der Ansprüche 1-2, welche ein Nahrungsergänzungsmittel ist.

4. Die Zusammensetzung nach einem der Ansprüche 1-3, welche eine Tablette von 0,6-1,6 g ist.

5. Die Zusammensetzung nach einem der Ansprüche 1-4, wobei 44-60 Gew.-% der Partikel des L-Lysins eine Partikelgröße ≥ 400 µm haben und 68-78 Gew.-% der Partikel eine Partikelgröße ≥ 250 µm haben.

6. Die Zusammensetzung nach einem der Ansprüche 1-5, wobei 45-51 Gew.-% der Partikel des L-Lysins eine Partikelgröße ≥ 400 µm haben und 70-76 Gew.-% der Partikel eine Partikelgröße ≥ 250 µm haben.

7. Die Zusammensetzung nach einem der Ansprüche 1-6, wobei die Menge an Methylsulfonylmethan im Bereich von 17 bis 22 Gew.-% bezogen auf das Gewicht der Kernzusammensetzung der Tablette liegt; wobei die Menge an L-Lysin oder ein Salz davon im Bereich von 21 bis 27 Gew.-% bezogen auf das Gewicht der Kernzusammensetzung der Tablette liegt; und wobei die Menge an Eisen(II)-Fumarat im Bereich von 2 bis 8 Gew.-% bezogen auf das Gewicht der Kernzusammensetzung der Tablette liegt.

8. Die Zusammensetzung nach einem der Ansprüche 1-7, weiterhin umfassend Biotin als Wirkstoff in einer Menge, die im Bereich von 0,001 bis 0,01 Gew.-% bezogen auf das Gewicht der Kernzusammensetzung der Tablette liegt.

9. Die Zusammensetzung nach einem der Ansprüche 1-8, wobei der Schmierstoff Magnesiumstearat ist.

10. Die Zusammensetzung nach einem der Ansprüche 1-9, wobei das Zerfallsbeschleuniger Croscarmellose-Natrium ist.

11. Die Zusammensetzung nach einem der Ansprüche 1-10, wobei die Menge an Methylsulfonylmethan im Bereich von 21 bis 22 Gew.-% bezogen auf das Gewicht der Kernzusammensetzung der Tablette liegt, die Menge an L-Lysin oder ein Salz davon im Bereich von 26 bis 27 Gew.-% bezogen auf das Gewicht der Kernzusammensetzung der Tablette liegt, die Menge an Eisen(II)-Fumarat im Bereich von 6 Gew.-% bezogen auf das Gewicht der Kernzusammensetzung der Tablette liegt, die Menge an Ascorbinsäure im Bereich von 3 bis 4 Gew.-% bezogen auf das Gewicht der Kernzusammensetzung der Tablette liegt, die Menge an Biotin im Bereich von 0,005 bis 0,006 Gew.-% bezogen auf das Gewicht der Kernzusammensetzung der Tablette liegt, und die Menge an Mikrokristalline Cellulose im Bereich von 35-40 Gew.-% bezogen auf das Gewicht der Kernzusammensetzung der Tablette liegt.

12. Die Zusammensetzung nach einem der Ansprüche 1-11, welche weiterhin eine äußere Beschichtung umfasst.

13. Eine Zusammensetzung wie in einem der Ansprüche 1-12 definiert, zur Verwendung bei der Behandlung von Haarausfall und/oder zur Stimulation des Haarwachstums bei einem Menschen, der es braucht.

14. Die Zusammensetzung zur Verwendung nach Anspruch 13, wobei der Mensch eine Frau ist.

## Revendications

1. Une composition pouvant être administrée par voie orale sous forme d'un comprimé pouvant être obtenu par compression directe, qui comprend une quantité thérapeutiquement efficace d'une combinaison d'ingrédients actifs et d'excipients acceptables comestibles,
dans laquelle,
la quantité totale des ingrédients actifs est égale à ou supérieure à 50 % du poids de la composition de noyau de comprimé ;
la combinaison d'ingrédients actifs comprend au moins :
a) du méthylsulfonylméthane, qui est présent dans une quantité dans l'intervalle allant de 5 à 40 % en poids par rapport au poids de la composition de noyau de comprimé ;
b) de la l-lysine ou un sel de celle-ci sous forme de particules, qui est présent(e) dans une quantité dans l'intervalle allant de 10 à 40 % en poids par rapport au poids de la composition de noyau de comprimé, et dans laquelle 40-70 % en poids des particules ont une taille des particules ≥ 400 µm et 60-85 % en poids des particules ont une taille des particules ≥ 250 µm ;
c) du fumarate ferreux qui est présent dans une quantité dans l'intervalle allant de 2 à 20% en poids par rapport au poids total de la composition de noyau de comprimé , et
l'excipient acceptable comestible comprend :
d) de la cellulose microcristalline sous forme de particules, qui est présente dans une quantité dans l'intervalle allant de 20 à 50 % en poids par rapport au poids de la composition de noyau de comprimé, et dans laquelle jusqu'à 5 % en poids des particules ont une taille des particules ≥ 400 µm, au moins 10 % en poids des particules ont une taille des particules ≥ 250 µm ; et au moins 50 % en poids des particules ont une taille des particules ≥ 150 µm ;
e) au moins un agent lubrifiant ; et
f) au moins un agent de désagrégation.

2. La composition selon la revendication 1, comprenant en outre un autre diluant.

3. La composition selon l'une quelconque des revendications 1-2, qui est un supplément nutritionnel.

4. La composition selon l'une quelconque des revendications 1-3, qui est un comprimé de 0,6-1,6 g.

5. La composition selon l'une quelconque des revendications 1-4, dans laquelle 44-60% en poids des particules de la L-lysine ont une taille des particules ≥ 400 µm et 68-78 % en poids des particules ont une taille des particules ≥ 250 µm.

6. La composition selon l'une quelconque des revendications 1-5, dans laquelle 45-51% en poids des particules de la L-lysine ont une taille des particules ≥ 400 µm et 70-76 % en poids des particules ont une taille des particules ≥ 250 µm.

7. La composition selon l'une quelconque des revendications 1-6, dans laquelle la quantité de méthylsulfonylméthane est dans l'intervalle allant de 17 à 22 % en poids par rapport au poids de la composition de noyau de comprimé ; la quantité de L-lysine ou d'un sel de celle-ci est dans l'intervalle allant de 21 à 27 % en poids par rapport au poids de la composition de noyau de comprimé ; et la quantité de fumarate ferreux est dans l'intervalle allant de 2 à 8 % en poids par rapport au poids de la composition de noyau de comprimé.

8. La composition selon l'une quelconque des revendications 1-7, comprenant en outre de la biotine comme ingrédient actif dans une quantité qui est dans l'intervalle allant de 0,001 à 0,01 % en poids par rapport au poids de la composition de noyau de comprimé.

9. La composition selon l'une quelconque des revendications 1-8, dans laquelle le lubrifiant est le stéarate de magnésium.

10. La composition selon l'une quelconque des revendications 1-9, dans laquelle l'agent de désagrégation est le croscarmellose de sodium.

11. La composition selon l'une quelconque des revendications 1-10, dans laquelle la quantité de méthylsulfonylméthane est dans l'intervalle allant de 21 à 22 % en poids par rapport au poids de la composition de noyau de comprimé, la quantité de L-lysine ou d'un sel de celle-ci est dans l'intervalle allant de 26 à 27 % en poids par rapport au poids de la composition de noyau de comprimé, la quantité de fumarate ferreux est dans l'intervalle de 6 % en poids par rapport au poids de la composition de noyau de comprimé, la quantité d'acide ascorbique est dans l'intervalle allant de 3 à 4 % en poids par rapport au poids de la composition de noyau de comprimé, la quantité de biotine est dans l'intervalle allant de 0,005 à 0,006 % en poids par rapport au poids de la composition de noyau de comprimé, et la quantité de cellulose microcristalline est dans l'intervalle de 35-40 % en poids par rapport au poids de la composition de noyau de comprimé.

12. La composition selon l'une quelconque des revendications 1-11, qui comprend en outre un revêtement extérieur.

13. Une composition telle que définie dans l'une quelconque des revendications 1-12, pour l'utilisation dans le traitement de la perte de cheveux et/ou la stimulation de sa croissance chez un individu qui en a besoin.

14. La composition pour l'utilisation selon la revendication 13, où le sujet est une femme.
